(19) Europäisches Patentamt
European Patent Office
Office européen des brevets

(11) EP 0 995 429 A1

# (12) EUROPÄISCHE PATENTANMELDUNG

(43) Veröffentlichungstag:
**26.04.2000 Patentblatt 2000/17**

(51) Int. Cl.[7]: **A61K 7/48**

(21) Anmeldenummer: **99117473.1**

(22) Anmeldetag: **10.09.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **25.09.1998 DE 19844054**

(71) Anmelder:
**Beiersdorf Aktiengesellschaft**
**20245 Hamburg (DE)**

(72) Erfinder:
- **Dörschner, Albrecht**
  **20146 Hamburg (DE)**
- **Nissen, Bente**
  **20253 Hamburg (DE)**

## (54) Verwendung von Silikonemulgatoren und anderen grenzflächenaktiven Agentien zur Verstärkung des Lichtschutzfaktors und/oder der UV-A-Schutzleistung kosmetischer oder dermatologischer Lichtschutzmittel

(57) Kosmetische oder dermatologische W/O-Emulsionen, welche höchstens 0,1 Gew.-% an einer oder mehreren Lichtschutzfiltersubstanzen aus der Gruppe der Dibenzoylmethanderivate, Benzylidencampherderivate, Triazinderivate, Salicylsäurederivate, Benzophenonderivate und Zimtsäurederivate enthalten und bevorzugt gänzlich frei von diesen Substanzen sind, ferner

(a) ein oder mehrere kosmetisch oder pharmazeutisch unbedenkliche anorganische Pigmenten enthalten, welche bevorzugt oberflächlich hydrophobiert sind, ferner enthaltend
(b) eine oder mehrere grenzflächenaktive Substanzen enthalten, und sich
(c) durch einen Gehalt an Siliconemulgatoren auszeichnen, und
(d) gewünschtenfalls ein oder mehrere Tocopherolderivate enthalten.

EP 0 995 429 A1

## Beschreibung

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. Man hat lange Zeit fälschlicherweise angenommen, daß die langweilige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Str„ahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch „kommt es zu chronischen lichtbedingten Hautveränderungen - die Haut „altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD ≡ **i**mmediate **p**igment **d**arkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, die insbesondere auch im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen

zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Bekannte und vorteilhafte Lichtschutzfiltersubstanzen sind Dibenzoylmethanderivate, beispielsweise 5-Isopropyldibenzoylmethan (CAS-Nr. 63250-25-9), welches sich durch die Struktur

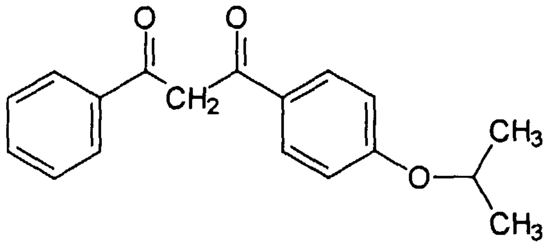

auszeichnet und von Merck unter der Marke Eusolex® 8020 verkauft wird, sowie das 4-(tert.-Butyl)-4'-methoxydibenzoylmethan (CAS-Nr. 70356-09-1), welches sich durch die Struktur

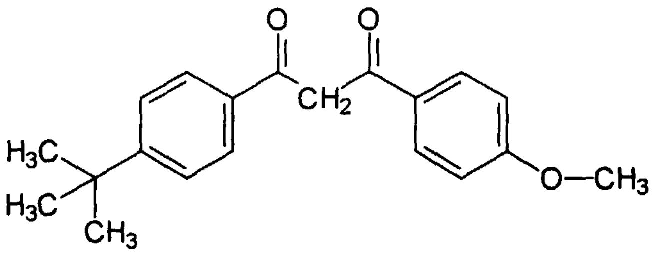

auszeichnet, und von Givaudan unter der Marke Parsol® 1789 verkauft wird. Gerade in Kombination mit anderen als Festkörper vorliegenden Substanzen ist ihre Einsatzkonzentration jedoch begrenzt. Es bereitet daher gewisse formulierungstechnische Schwierigkeiten, höhere Lichtschutzfaktoren zu erzielen.

Eine weitere vorteilhafte Lichtschutzfiltersubstanz ist der 4-Methylbenzylidencampher, welcher sich durch die Struktur

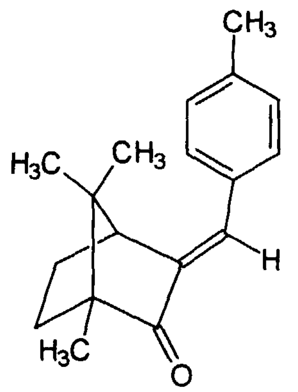

auszeichnet und von Merck unter der Marke Eusolex® 6300 verkauft wird. 4-Methylbenzylidencampher ist eine äußerst vorteilhafte, unter Normalbedingungen als Festkörper vorliegende Lichtschutzfiltersubstanz und zeichnet sich an sich durch gute UV-Filtereigenschaften aus. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Zwischenzeitlich wurden von verschiedenen Autoren auch andere UV-Filtersubstanzen beschrieben, welche das Strukturmotiv

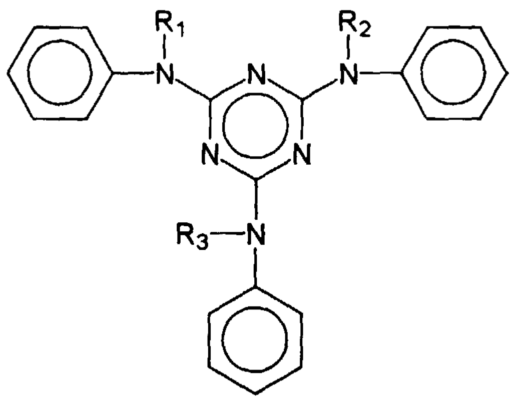

aufweisen.

Die teilweise vorstehend genannten Verbindungen, welche als Lichtschutzmittel für kosmetische und dermatologische Lichtschutzformulierungen eingesetzt werden, zeichnen sich, wie gesagt, an sich durch gute Lichtschutzwirkung aus. Sie haben jedoch den Nachteil, daß es bisweilen schwierig ist, sie in befriedigender Weise solchen Formulierungen einzuverleiben.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte „Ozonloch" zum einen zuverlässige Angaben des Herstellers zum Lichtschutzfaktor, zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es eine weitere Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Es wäre bevorzugt, ganz auf „chemische" Lichtschutzfiltersubstanzen, d.h., solche, deren UV-Filterleistung auf Absorption der Lichtquanten durch geeignete Chromophore beruht, gänzlich zu verzichten. Die einzige sinnvolle

Alternative zu solchen „chemischen" Lichtschutzfiltersubstanzen sind Mikropigmente, die im Fachjargon auch UV-Blokker genannt werden. Die UV-Filterleistung solcher Pigmente beruht in erster Linie auf Reflexion bzw. Streuung des eingestrahlten ultravioletten Lichtes.

Ein hoher LSF in Sonnenschutzprodukten ohne chemische Filter ist nur durch die Einarbeitung hoher Mengen an Micropigmenten zu erreichen. Dies ist nicht immer erwünscht, da entsprechende Zubereitungen Gefahr laufen, kosmetisch unelegant zu wirken oder gar ein unangenehmes Hautgefühl verursachen.

Es war daher überraschend und für den Fachmann nicht vorauszusehen, daß kosmetische oder dermatologische W/O-Emulsionen, welche höchstens 0,1 Gew.-% an einer oder mehreren Lichtschutzfiltersubstanzen aus der Gruppe der Dibenzoylmethanderivate, Benzylidencampherderivate, Triazinderivate, Salicylsäurederivate, Benzophenonderivate und Zimtsäurederivate enthalten und bevorzugt gänzlich frei von diesen Substanzen sind, ferner

(a) ein oder mehrere kosmetisch oder pharmazeutisch unbedenkliche anorganische Pigmenten enthalten, welche bevorzugt oberflächlich hydrophobiert sind, ferner enthaltend

(b) eine oder mehrere grenzflächenaktive Substanzen der der allgemeinen Formel (1)

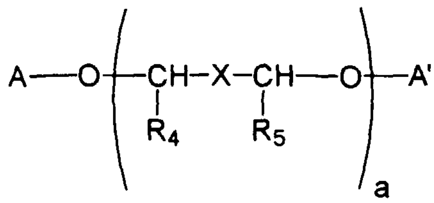
,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

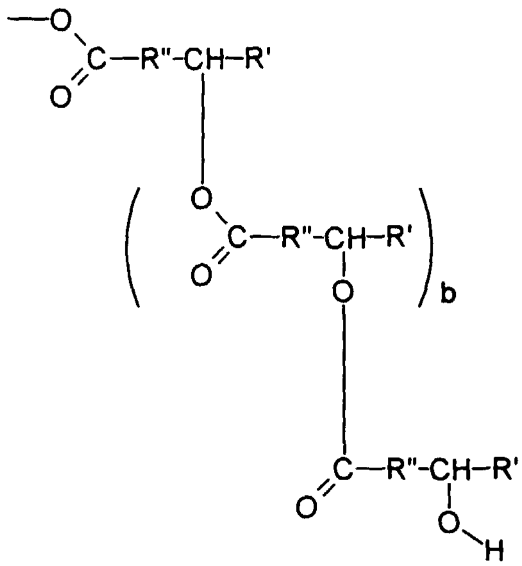
,

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

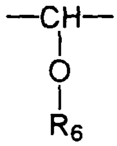

- darstellt,
- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen, und sich

(c) durch einen Gehalt an Siliconemulgatoren auszeichnen, und
(d) gewünschtenfalls ein oder mehrere Tocopherolderivate enthalten,

besonders vorteilhafte Eigenschaften aufweisen.

Es war insbesondere überraschend, daß erfindungsgemäße Zubereitungen sich durch einen gegenüber den Erwartungen deutlich erhöhten Lichtschutzfaktor und eine erhöhte UV-A-Schutzleistung auszeichnen.

Gegenstand der Erfindung ist demgemäß auch die Verwendung von Gemischen aus

(b) einer oder mehreren grenzflächenaktiven Substanzen der der allgemeinen Formel (1)

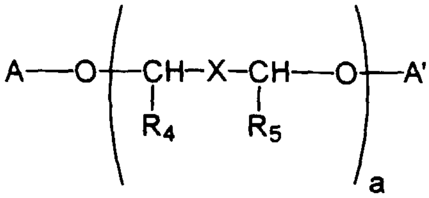

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

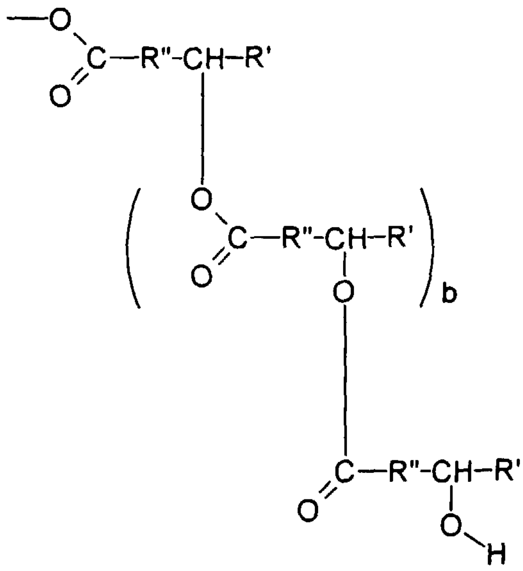

,

wobei R' gewählt wird aus der Gruppe der verzweigten und unverzweigten Alkylgruppen mit 1 bis 20 Kohlenstoffatomen und R'' gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

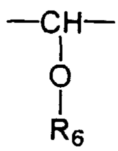

- darstellt,
- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen,

(c) einem oder mehreren Siliconemulgatoren
(d) gewünschtenfalls einem oder mehreren Tocopherolderivaten,

zur Erhöhung des Lichtschutzfaktors und/oder zur Erhöhung der UV-A-Schutzleistung kosmetischer oder dermatologischer Zubereitungen, welche

(a) ein oder mehrere kosmetisch oder pharmazeutisch unbedenkliche anorganische

Pigmenten enthalten, welche bevorzugt oberflächlich hydrophobiert sind, und welche höchstens 0,1 Gew.-% an einer oder mehreren Lichtschutzfiltersubstanzen aus der Gruppe der Dibenzoylmethanderivate, Benzylidencampherderivate, Triazinderivate, Salicylsäurederivate, Benzophenonderivate und Zimtsäurederivate enthalten, und welche bevorzugt gänzlich frei von diesen Substanzen sind.

Erfindungsgemäß können die Siliconemulgatoren vorteilhaft aus der Gruppe grenzflächenaktive Substanzen aus der Gruppe der Alkylmethiconcopolyole und/oder Alkyl-Dimethiconcopolyole gewählt werden

Ein Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL ® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th.Goldschmidt AG unter der Warenbezeichnung ABIL ® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß verwendeten Siliconemulgatoren in den erfindungsgemäßen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

In den Substanzen der allgemeinen Strukturformel

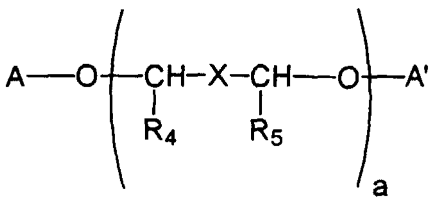

können A, A', $R_4$ und $R_5$ vorteilhaft Wasserstoffatome darstellen, werden aber ebenfalls vorteilhaft aus der Gruppe Methyl-, Ethyl-, Propyl- und Isopropyl-, Myristoyl-, Palmitoyl-, Stearoyl- und Eicosoyl- gewählt, sowie aus der Gruppe, die sich durch die chemischen Strukturen .

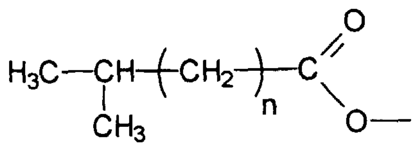

wobei n Zahlen von 10 bis 20 annimmt, von diesen bevorzugt der Isostearoylrest, bzw.

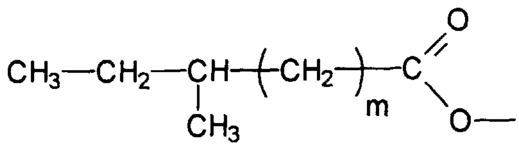

wobei m Zahlen von 9 bis 19 annimmt.

Als vorteilhaft haben sich erfindungsgemäße lichtschutzwirksame Wirkstoffkombinationen erwiesen, in welchen die grenzflächenaktiven Substanzen der der allgemeinen Formel (1) Monoglycerinester, Diglycerinester, Triglycerinester bzw. Tetraglycerinester darstellen und beispielsweise Monoester der Isostearinsäure sind, insbesondere vorteilhaft ist das Tetraglycerinmonoisostearat, welches analog zur CTFA-Nomenklatur auch Polyglyceryl-4-Isostearat genannt wird.

Solche Isostearinsäureester sind beispielsweise erhältlich unter der Produktbezeichnung „Isolan GI 34" der Gesellschaft Henkel Goldschmidt Chemical Corp.

Als besonders vorteilhaft haben sich auch erfindungsgemäße lichtschutzwirksame Wirkstoffkombinationen erwiesen, in welchen die grenzflächenaktiven Substanzen der der allgemeinen Formel (1) Monoglycerinester, Diglycerinester, Triglycerinester bzw. Tetraglycerinester darstellen und Diester der Isostearinsäure sind, insbesondere bevorzugt ist das Triglycerindiisostearat, welches analog zur CTFA-Nomenklatur auch Polyglyceryl-3-Diisostearat genannt wird.

Solche Isostearinsäureester sind beispielsweise erhältlich unter der Produktbezeichnung „Lameform TGI" der Gesellschaft Henkel KGaA.

Als ebenfalls besonders vorteilhaft haben sich erfindungsgemäße lichtschutzwirksame Wirkstoffkombinationen erwiesen, in welchen die grenzflächenaktiven Substanzen der der allgemeinen Formel (1) Monoglycerinester, Diglycerinester, Triglycerinester bzw. Tetraglycerinester darstellen und Gemische aus Monoestern und Diestern der Isostearinsäure enthalten, insbesondere bevorzugt dabei sind etwa äquimolare Gemische, wie beispielsweise das

Diglycerinsesquiisostearat, welches analog zur CTFA-Nomenklatur auch Polyglyceryl-2-Sesquiisostearat genannt wird.

Solche Isostearinsäureester sind beispielsweise erhältlich unter der Produktbezeichnung „Hostacerin DGI" der Gesellschaft Hoechst AG.

Als besonders vorteilhaft haben sich erfindungsgemäße lichtschutzwirksame Wirkstoffkombinationen erwiesen, in welchen die grenzflächenaktiven Substanzen der der allgemeinen Formel (1) die Polyesterreste von der Hydroxystearinsäure abgeleitet sind, insbesondere vorteilhaft das „Polyglyceryl-2-Polyhydroxystearat", welches unter den Regstriernummern 156531-21-4 bzw. 144470-58-6 in den „Chemical Abstracts" abgelegt ist, und welcher beispielsweise unter der Warenbezeichnung DEHYMULS® PGPH von der Henkel KGaA erhältlich ist.

Ein Beispiel für besonders vorteilhaft im Sinne der vorliegenden Erfindung zu verwendende grenzflächenaktiven Substanzen ist ferner das PEG-30-Dipolyhydroxystearat, welches von der Gesellschaft ICI Surfactants unter der Warenbezeichnung ARLACEL® P135 verkauft wird.

Erfindungsgemäß können diese grenzflächenaktiven Substanzen der Formel (1) in Konzentrationen von 0,005 bis 50 Gew.%, bezogen auf das Gesamtgewicht der Zubereitungen, vorliegen. Dabei werden Konzentrationen von 0,5 - 10 Gew.-%, insbesondere 1,0 - 5 Gew.-%, bevorzugt.

Die gewünschtenfalls in den Zubereitungen gemäß der Erfindung einzusetzenden Tocopherole, auch Vitamin E genannt, leiten sich vom Grundkörper Tocol ((2-Methyl-2-(4,8,12-trimethyltridecyl)chroman-6-ol) ab und sind durch folgende Strukturen gekennzeichnet:

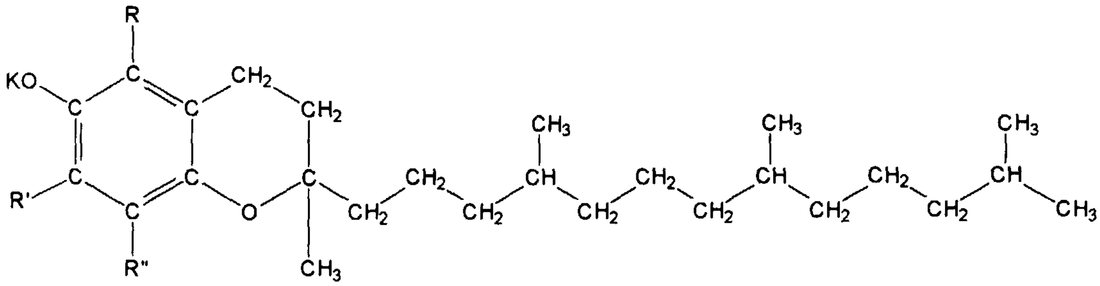

Dab ei stellt K entweder H oder einen Acylrest dar, R, R' und R" bedeuten unabhängig voneinander H oder eine Methylgruppe, z.B.:

| | |
|---|---|
| R = R' = R" = K = H | : Tocol |
| R = R' = R" = Methyl, K = H | : α-Tocopherol |
| R = R" = Methyl, R' = K = H | : β-Tocopherol |

R = R' = R" = Methyl, K = 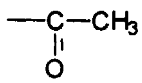 : α-Tocopherolacetat

Dem natürlich am häufigsten vorkommenden und bedeutendsten α-Tocopherol kommt die Konfiguration 2R,4'R,8'R zu. Es wird gelegentlich auch RRR-α-Tocopherol genannt.

Die erfindungsgemäß besonders vorteilhaften Tocopherolderivate sind das α-Tocopherol und seine Ester, insbesondere das α-Tocopherylacetat.

Ebenfalls vorteilhaft sind Tocopherylglycoside, insbesondere Tocopherylglycoside der allgemeinen Formel

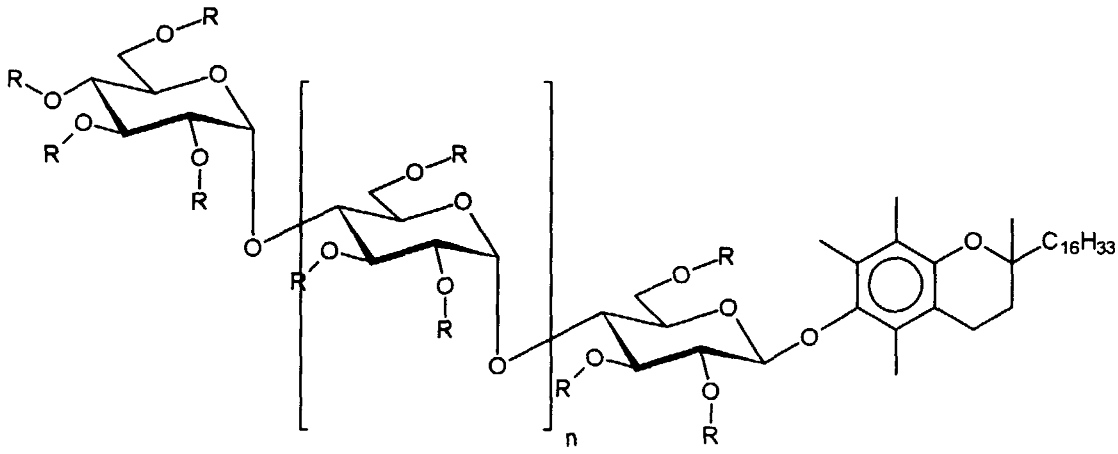

und/oder der allgemeinen Formel

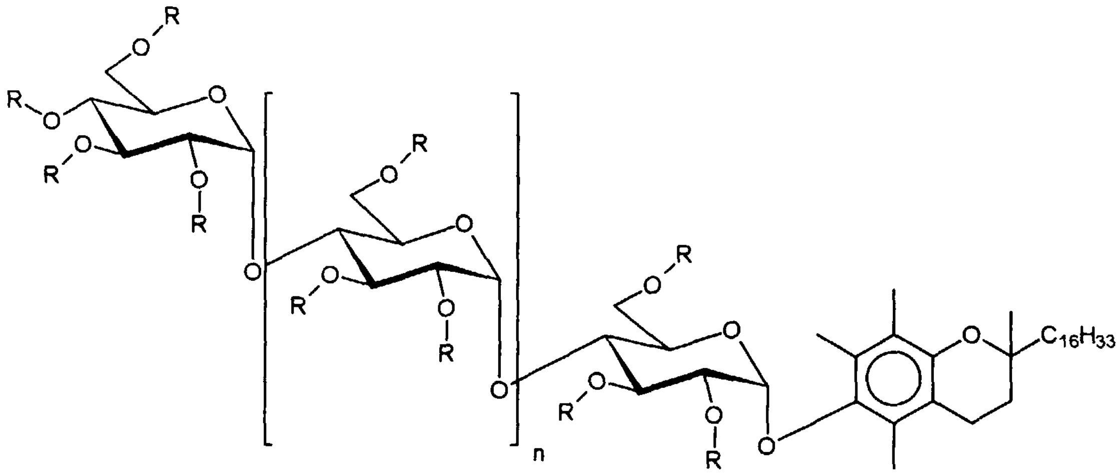

wo bei n Werte von 0 - 6 annehmen kann und wobei R stellvertretend steht für die Gruppe bestehend aus den Resten H, verzweigtem und unverzweigtem Alkyl von 1 - 18 C-Atomen, verzweigtem und unverzweigtem Acyl von 1 - 18 C-Atomen, und wobei R innerhalb eines Moleküls in allen Positionen der Glycosylgruppen gleich sein kann, aber auch innerhalb eines Moleküls unterschiedliche Bedeutung annehmen kann, dergestalt, daß innerhalb eines Moleküls beliebige Kombinationen der steilvertretenen Reste gewählt werden dürfen,

Dabei ist es vorteilhaft, die den erfindungsgemäßen Tocopheryglycosiden zugrundeliegende Tocopheroleinheit aus allen natürlichen oder synthetisch zugängigen Tocopherolkörpern zu wählen. Erfindungsgemäß besonders vorteilhaft ist, als zugrundeliegende Tocopheroleinheit DL-α-Tocopherol in seiner natürlichen Konfiguration zu wählen. Ganz besonders vorteilhaft stellt R in allen Substitutionspositionen H dar.

Die bevorzugten Verbindungen tragen folgende Bezeichnungen:

| | |
|---|---|
| n= 0: Tocopheryl-β-D-maltosid | bzw. Tocopheryl-α-D-maltosid |
| n= 1: Tocopheryl-β-D-maltotriosid | bzw. Tocopheryl-α-D-maltotriosid |
| n= 2: Tocopheryl-β-D-maltotetraosid | bzw. Tocopheryl-α-D-maltotetraosid |
| n= 3: Tocopheryl-β-D-maltopentaosid | bzw. Tocopheryl-α-D-maltopentaosid |

(fortgesetzt)

| | |
|---|---|
| n= 4: Tocopheryl-β-D-maltohexaosid | bzw. Tocopheryl-α-D-maltohexaosid |
| n= 5: Tocopheryl-β-D-maltoheptaosid | bzw. Tocopheryl-α-D-maltohexaosid |
| n= 6: Tocopheryl-β-D-maltooctaosid | bzw. Tocopheryl-α-D-maltooctaosid |
| n= 7: Tocopheryl-β-D-maltoenneaosid | bzw. Tocopheryl-α-D-maltoenneaosid |
| n= 8: Tocopheryl-β-D-maltodekaosid | bzw. Tocopheryl-α-D-maltodekaosid |

Im allgemeinen sind sowohl die Tocopheryl-α-Glycoside als auch die Tocopheryl-β-Glycoside erfindungsgemäß vorteilhaft zu verwenden. Besonders vorteilhaft sind die β-Glycoside. Auch Gemische aus α- und β-Glycosiden können erfindungsgemäß vorteilhaft sein. Besonders vorteilhaft ist, wenn n den Wert 1 annimmt. Ganz besonders vorteilhaft stellt R in allen Substitutionspositionen H dar.

Es ist vorteilhaft, die Konzentrationen des oder der Tocopherolderivate aus dem Bereich von 0,001 - 10 Gew.-%, bezogen auf das Gesamtgewicht der Formulierung, zu wählen.

Erfindungsgemäße kosmetische und dermatologische Zubereitungen enthalten anorganische Pigmente, welche röntgenamorph oder nichtröntgenamorph sind, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nichtröntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handeisbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0 Gew.-% gewählt, bezogen auf das Gesamtgewicht der Zubereitungen.

Die erfindungsgemäßen kosmetischen und/oder dermatologischen Lichtschutzformulierungen können wie

üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. $ZnO$, $ZnSO_4$), Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane, Cetyldimethicone sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Besonders vorteilhaft kann die Ölphase ferner einen Gehalt an cyclischen oder linearen Silikonölen aufweisen oder vollständig aus solchen Ölen bestehen Es wird bevorzugt, die Ölphase der erfindungsgemäßen Zubereitungen aus der Gruppe der cyclischen und/oder linearen Silicone zu wählen, welche im Rahmen der vorliegenden Offenbarung auch als „Siliconöle" bezeichnet werden. Solche Silicone oder Siliconöle können als Monomere vorliegen, welche in der Regel durch Strukturelemente charakterisiert sind, wie folgt:

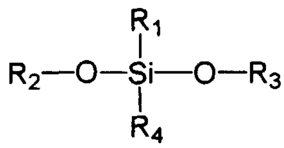

Als erfindungsgemäß vorteilhaft einzusetzenden linearen Silicone mit mehreren Siloxyleinheiten werden im allgemeinen durch Strukturelemente charakterisiert wie folgt:

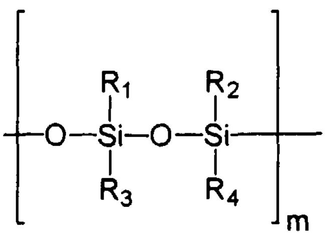

,

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt

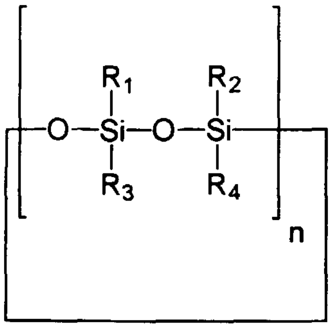

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

<u>Beispiel 1</u>

| | Gew.-% |
|---|---|
| Polyglyceryl-2 Polyhydroxystearat | 1,50 |
| Cetyldimethicone Copolyol | 4,50 |
| Paraffinum liquidum | 3,00 |
| Cyclomethicon | 5,00 |
| $C_{12-15}$-Alkylbenzoate | 2,00 |
| Isohexadecan | 2,00 |
| $TiO_2$ | 2,00 |
| $MgSO_4$ | 1,00 |

(fortgesetzt)

| | Gew.-% |
|---|---|
| Glycerin | 5,00 |
| Ethanol | 2,00 |
| Panthenol | 1,00 |
| Tocopherylacetat | 0,50 |
| Phenoxyethanol | 0,50 |
| Wasser | ad 100,00 |

Beispiel 2

| | Gew.-% |
|---|---|
| Polyglyceryl-2 Polyhydroxystearat | 1,50 |
| Cetyldimethicone Copolyol | 4,50 |
| Paraffinum liquidum | 6,00 |
| Phenyltrimethicone | 2,00 |
| $C_{12-15}$-Alkylbenzoate | 5,00 |
| Isohexadecan | 5,00 |
| $TiO_2$ | 12,00 |
| $MgSO_4$ | 1,00 |
| Glycerin | 5,00 |
| Ethanol | 2,00 |
| Sodium Corn Starch Octenylsuccinate | 1,00 |
| Panthenol | 1,00 |
| Tocopherylacetate | 0,50 |
| Phenoxyethanol | 0,50 |
| Wasser | ad 100,00 |

Beispiel 3

| | Gew.-% |
|---|---|
| Polyglyceryl-2 Polyhydroxystearat | 1,50 |
| Cetyldimethicone Copolyol | 4,50 |
| Paraffinum liquidum | 6,00 |
| $C_{12-15}$-Alkylbenzoate | 5,00 |

(fortgesetzt)

| | Gew.-% |
|---|---|
| Isohexadecan | 5,00 |
| Cetyl Dimethicone | 1,00 |
| Dimethicone | 2,00 |
| Titanium Dioxide | 12,00 |
| Zinc Oxide | 2,00 |
| $MgSO_4$ | 1,00 |
| Glycerin | 5,00 |
| Ethanol | 2,00 |
| Sodium Corn Starch Octenylsuccinate | 1,00 |
| Panthenol | 1,00 |
| Tocopheryl Acetate | 0,50 |
| Phenoxyethanol | 0,50 |
| Wasser | ad 100,00 |

Beispiel 4

| | Gew.-% |
|---|---|
| Polyglyceryl-2-Polyhydroxystearat | 1,50 |
| Cetyldimethicone Copolyol | 4,50 |
| Paraffinum liquidum | 6,00 |
| Cyclomethicone | 6,00 |
| $C_{12-15}$-Alkyl Benzoate | 5,00 |
| Isohexadecan | 5,00 |
| $TiO_2$ | 8,00 |
| ZnO | 8,00 |
| $MgSO_4$ | 1,00 |
| Glycerin | 5,00 |
| Ethanol | 2,00 |
| Sodium Corn Starch Octenylsuccinate | 1,00 |
| Panthenol | 1,00 |
| Tocopherylacetat | 0,50 |
| Phenoxyethanol | 0,50 |
| Wasser | ad 100,00 |

Beispiel 5

| | Gew.-% |
|---|---|
| Polyglyceryl-2-Polyhydroxystearate | 1,50 |
| Cetyldimethicone Copolyol | 4,50 |
| Paraffinum liquidum | 6,00 |
| Cetyldimethicone | 1,00 |
| Dimethicone | 2,00 |
| Cyclomethicone | 3,00 |
| $C_{12-15}$ Alkylbenzoate | 3,00 |
| Isohexadecan | 5,00 |
| $TiO_2$ | 8,00 |
| ZnO | 8,00 |
| $MgSO_4$ | 1,00 |
| Glycerin | 5,00 |
| Ethanol | 2,00 |
| Sodium Corn Starch Octenylsuccinate | 1,00 |
| Panthenol | 1,00 |
| Tocopherylacetat | 0,50 |
| Phenoxyethanol | 0,50 |
| Wasser | ad 100,00 |

**Patentansprüche**

**1.** Kosmetische oder dermatologische W/O-Emulsionen, welche höchstens 0,1 Gew.-% an einer oder mehreren Lichtschutzfiltersubstanzen aus der Gruppe der Dibenzoylmethanderivate, Benzylidencampherderivate, Triazinderivate, Salicylsäurederivate, Benzophenonderivate und Zimtsäurederivate enthalten und bevorzugt gänzlich frei von diesen Substanzen sind, ferner

(a) ein oder mehrere kosmetisch oder pharmazeutisch unbedenkliche anorganische Pigmenten enthalten, welche bevorzugt oberflächlich hydrophobiert sind, ferner enthaltend
(b) eine oder mehrere grenzflächenaktive Substanzen der der allgemeinen Formel (1)

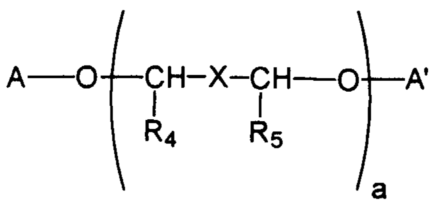

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

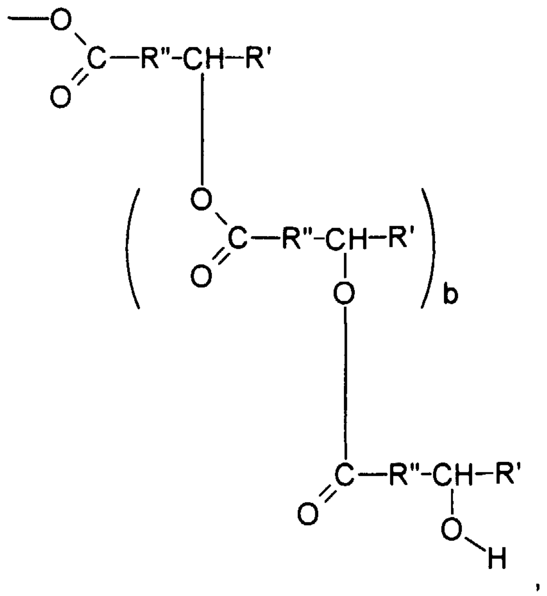

wobei R' gewählt wird aus der Gruppe der verzweigten und unverzweigten Alkylgruppen mit 1 bis 20 Kohlenstoffatomen und R'' gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

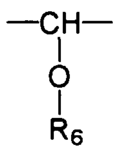

- darstellt,
- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen, und sich

(c) durch einen Gehalt an Siliconemulgatoren auszeichnen, und
(d) gewünschtenfalls ein oder mehrere Tocopherolderivate enthalten.

**2.** Verwendung von Gemischen aus

(b) einer oder mehreren grenzflächenaktiven Substanzen der der allgemeinen Formel (1)

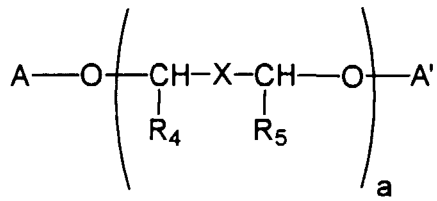

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

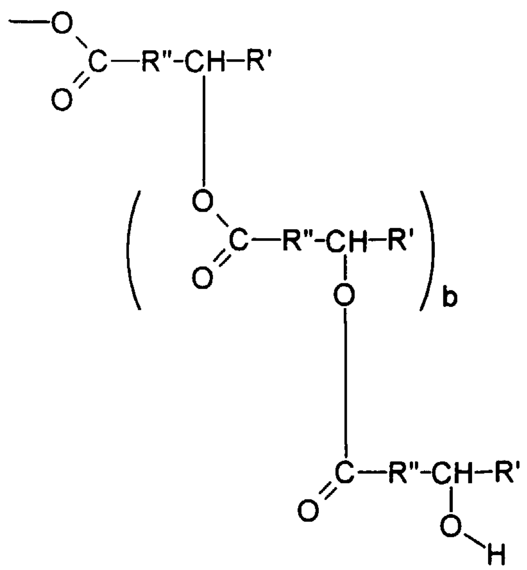

,

wobei R' gewählt wird aus der Gruppe der verzweigten und unverzweigten Alkylgruppen mit 1 bis 20 Kohlenstoffatomen und R'' gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

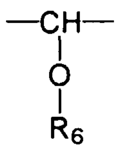

- darstellt,
- $R_4$ und $R_5$ unabhängig voneinander aus der Gruppe H, Methyl gewählt werden,
- $R_6$ gewählt wird aus der Gruppe H, sowie der verzweigten und unverzweigten, gesättigten und ungesättigten Alkyl- und Acylreste mit 1 - 20 Kohlenstoffatomen,

(c) einem oder mehreren Siliconemulgatoren

(d) gewünschtenfalls einem oder mehreren Tocopherolderivaten,

zur Erhöhung des Lichtschutzfaktors und/oder zur Erhöhung der UV-A-Schutzleistung kosmetischer oder dermatologischer Zubereitungen, welche

(a) ein oder mehrere kosmetisch oder pharmazeutisch unbedenkliche anorganische

Pigmenten enthalten, welche bevorzugt oberflächlich hydrophobiert sind, und welche höchstens 0,1 Gew.-% an einer oder mehreren Lichtschutzfiltersubstanzen aus der Gruppe der Dibenzoylmethanderivate, Benzylidencampherderivate, Triazinderivate, Salicylsäurederivate, Benzophenonderivate und Zimtsäurederivate enthalten, und welche bevorzugt gänzlich frei von diesen Substanzen sind.

**3.** W/O-Emulsionen nach Anspruch 1 oder Verwendung nach Anspruch 2, dadurch gekennzeichnet, daß der oder die Siliconemulgatoren aus der Gruppe der grenzflächenaktive Substanzen aus der Gruppe der Alkylmethiconcopolyole und/oder Alkyl-Dimethiconcopolyole gewählt werden.

**4.** W/O-Emulsionen nach Anspruch 1 oder Verwendung nach Anspruch 2, dadurch gekennzeichnet, daß der oder die Siliconemulgatoren aus der Gruppe Cetyl Dimethiconcopolyol, Cyclomethicon Dimethiconcopolyol und Laurylmethiconcopolyol gewählt wird.

**5.** W/O-Emulsionen nach Anspruch 1 oder Verwendung nach Anspruch 2, dadurch gekennzeichnet, daß die Gesamtmenge an Siliconemulgatoren in den kosmetischen oder dermatologischen Zubereitungen aus dem Bereich von 0,1 - 10,0 Gew.-%, bevorzugt 0,5 - 6,0 Gew.-% gewählt, bezogen auf das Gesamtgewicht der Zubereitungen, gewählt wird.

**6.** W/O-Emulsionen nach Anspruch 1 oder Verwendung nach Anspruch 2, dadurch gekennzeichnet, daß das oder die anorganischen Pigmente röntgenamorph oder nichtröntgenamorph sind und auf der Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden beruhen, besonders bevorzugt Pigmente auf der Basis von $TiO_2$ darstellen.

**7.** W/O-Emulsionen nach Anspruch 1 oder Verwendung nach Anspruch 2, dadurch gekennzeichnet, daß die Gesamtmenge an anorganischen Pigmenten, insbesondere hydrophoben anorganischen Mikropigmenten in den fertigen kosmetischen oder dermatologischen Zubereitungen aus dem Bereich von 0,1 - 30 Gew.-%, bevorzugt 0,1 - 10,0 Gew.-% gewählt wird, bezogen auf das Gesamtgewicht der Zubereitungen.

**8.** W/O-Emulsionen nach Anspruch 1 oder Verwendung nach Anspruch 2, dadurch gekennzeichnet, daß die Konzentrationen des oder der Tocopherolderivate aus dem Bereich von 0,001 - 10 Gew.-%, bezogen auf das Gesamtgewicht der Formulierung, gewählt wird.

Europäisches Patentamt

# EUROPÄISCHER RECHERCHENBERICHT

Nummer der Anmeldung
EP 99 11 7473

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | FR 2 759 583 A (OREAL)<br>21. August 1998 (1998-08-21)<br>* Beispiel 3 *<br>--- | | A61K7/48 |
| A | EP 0 858 800 A (OREAL)<br>19. August 1998 (1998-08-19)<br>* Beispiele 1,2 *<br>--- | | |
| A | DE 196 42 090 A (BEIERSDORF AG)<br>9. April 1998 (1998-04-09)<br>* Beispiele 13,14,20-22 *<br>----- | | |
| | | | RECHERCHIERTE SACHGEBIETE (Int.Cl.7) |
| | | | A61K |

Der vorliegende Recherchenbericht wurde für alle Patentansprüche erstellt

| Recherchenort | Abschlußdatum der Recherche | Prüfer |
|---|---|---|
| DEN HAAG | 10. Februar 2000 | Stienon, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 99 11 7473

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-02-2000

| Im Recherchenbericht angeführtes Patentdokument | Datum der Veröffentlichung | Mitglied(er) der Patentfamilie | Datum der Veröffentlichung |
|---|---|---|---|
| FR 2759583 A | 21-08-1998 | AU 706809 B | 24-06-1999 |
| | | AU 6105398 A | 08-09-1998 |
| | | BR 9805951 A | 31-08-1999 |
| | | CA 2251633 A | 20-08-1998 |
| | | CN 1217651 T | 26-05-1999 |
| | | EP 0893987 A | 03-02-1999 |
| | | WO 9835649 A | 20-08-1998 |
| | | JP 11507961 T | 13-07-1999 |
| EP 0858800 A | 19-08-1998 | FR 2758982 A | 07-08-1998 |
| | | AU 695027 A | 06-08-1998 |
| | | BR 9800660 A | 06-07-1999 |
| | | CA 2226393 A | 06-08-1998 |
| | | CN 1196238 A | 21-10-1998 |
| | | HU 9800246 A | 28-01-1999 |
| | | JP 10218727 A | 18-08-1998 |
| | | PL 324674 A | 17-08-1998 |
| DE 19642090 A | 09-04-1998 | WO 9815254 A | 16-04-1998 |
| | | EP 0930866 A | 28-07-1999 |

EPO FORM P0461

Für nähere Einzelheiten zu diesem Anhang : siehe Amtsblatt des Europäischen Patentamts, Nr.12/82